# EUROPEAN PATENT SPECIFICATION

(11) **EP 3 415 080 B1**
(45) Date of publication and mention of the grant of the patent: **01.11.2023**
(21) Application number: 17305704.3
(22) Date of filing: 12.06.2017
(51) Int. Cl.: A61B 5/369, A61B 5/00

(54) **WEARABLE DEVICE**
WEARABLE-VORRICHTUNG
DISPOSITIF VESTIMENTAIRE

(43) Date of publication of application: 19.12.2018
(73) Proprietor: DREEM, 75009 Paris (FR)
(72) Inventor: MERCIER, Hugo, 75010 Paris (FR); SOULET DE BRUGIERE, Quentin, 75001 Paris (FR); HERRERA, Martin, 92100 Boulogne-Billancourt (FR); KERBAUL, Camille, 75018 Paris (FR); EMERICH, Pierre, 75011 Paris (FR)
(74) Representative: Plasseraud IP

(56) References cited:
- GB-A- 2 516 275
- US-A1- 2014 257 073
- US-A1- 2017 042 439

## Description

The present invention relates to a wearable device.

It more particularly relates to a wearable device used to control the cerebral activity of a user during his sleep, by stimulating the cerebral activity and acquiring bioelectric physiological signal by using brain electrodes.

Bioelectric physiological signals that are generated by biological beings that can be measured and monitored. Electroencephalographs and electrocardiographs are examples of devices that are used to measure and monitor bio-signals generated by humans.

Measuring and analyzing bioelectrical physiological signals such as brainwave patterns can have a variety of practical applications. For example, wearable devices have been developed to control the cerebral activity of a user during his sleep to reinforce the beneficial effects of the sleep. Such wearable devices are meant to be worn on the user's head during a period of sleep. The wearable device usually comprises one or more electrodes and/or sensor to measure the bioelectrical physiological signals of the user.

Controlling the cerebral activity by measuring and acquiring bioelectrical physiological signals using a wearable device may be difficult due to challenges in obtaining the signals, especially during a period of sleep of the user.

To obtain optimal bioelectrical physiological signals, the electrodes and/or the sensor have to be in contact with the user's head, particularly with the skin of the user's head; the wearable device has to fit to the user's head although a head may have a plurality of sizes and shapes.

Secondly, the wearable device must be sufficiently comfortable to allow the person to sleep while the person is wearing it.

Actually, there is no such device.

GB 2 516 275 relates to an electroencephalography headset, but which is not adjustable to heads having different diameters, but rather provides several sizing components that are replaceable and available in varying lengths or widths to accommodate different head sizes.

The present invention aims to improve the situation.

In a first aspect, the invention relates to a wearable device according to claim 1.

It has been found that an adjustment element, as described in the present invention, may allow the wearable device to fit to any size and shape of a user's head. Then the electrode may be in contact with the user's head regardless the size of the head. Moreover, including an adjustment element around a great part of the device stretches evenly and exerts even pressure on wearers having different head sizes, which enhance the comfort of the wearable device when it is worn.

In another aspect, the side bands and the upper band are able to form a structure having a semicircular shape able to encircle at least a portion of a circumference of the user's head.

In another aspect, the second side band ends of the first and second side bands do not touch each other such that a portion of the adjustment element is accessible between the second side band ends of said first and second side bands.

In another aspect, the front band forms an angle between 30° and 50° with the side bands, preferably an angle of 40°.

In another aspect, wherein the front band ends are disposed at equidistance from first and second side band ends of the side band the front band ends are connected to.

In another aspect, the wearable device has a sideway generally Y-shaped.

In another aspect, the adjustment element comprises two ends, each end being attached to a cleat disposed at the first end of first and second side bands.

In another aspect, the adjustment element is able to extend along its length when the wearable device is pulled tight on the user's head.

In another aspect, the side bands are made with a flexible material such that the side bands are able to adapt to the shape of the user's head.

In another aspect, an outer surface of the side bands is coated with a rigid film.

In another aspect, each electrode is disposed on a support placed at the second side band end of each side band such that the electrode is in contact with the rear part of the user's head when the wearable device is worn.

In another aspect, the electrodes are attached to the side bands by a plug made of conductive material.

In another aspect, the support is comprised in the electrode.

In another aspect, the upper band comprises embedded electronics.

In another aspect, the adjustment element is an elastic ribbon.

Other features and advantages of the invention will be apparent from reading the following detailed description of example embodiments and from examining the attached drawings, in which:
- figure 1 is a side view of a wearable device comprising an adjustment element,
- figure 2 is a rear view of a wearable device comprising an adjustment element,
- figure 3 illustrates the assembly of the adjustment element inside the wearable device,
- figure 4 shows an internal view of the wearable adjustment.

Below is a detailed description of several embodiments of the invention, with examples and with references to the drawings.

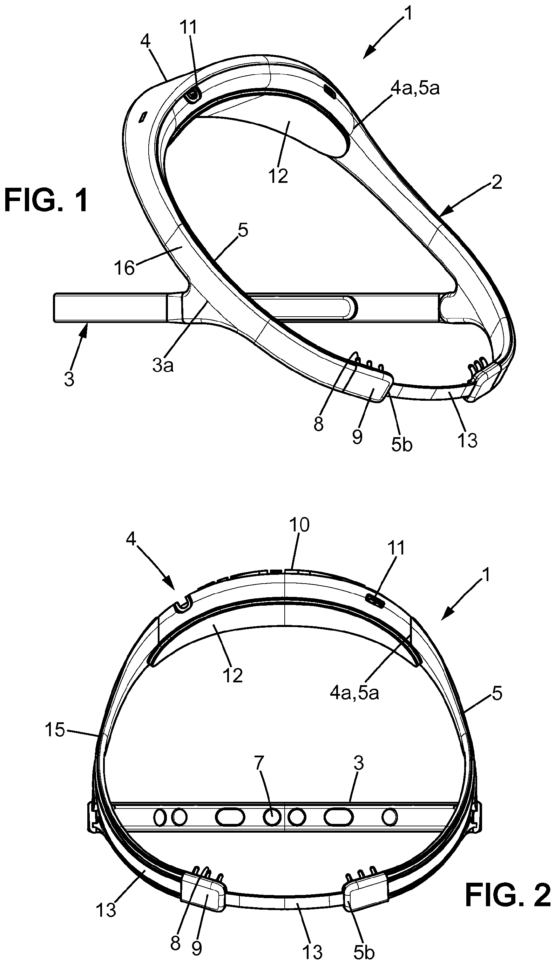

Figures 1 and 2 show a wearable device 1 according to an exemplary aspect of the invention.

The wearable device 1 is able to be worn by a user, especially during a period of sleep of the user.

The wearable device 1 is particularly able to be worn on the head of the user.

For this purpose, the wearable device 1 comprises a band assembly 2. The band assembly 2 is able to encircle at least one part of the user's head such that the band assembly 2 is maintained on the user's head during his sleep. The band assembly 2 is able to encircle at least a portion of a circumference of the user's head, especially at least a half of a circumference of the user's head, or even an entire diameter of the user's head.

In the exemplary embodiment represented figures 1-4, the band assembly 2 comprises several bands 3, 4, 5. The band assembly 2 particularly comprises four bands. For example, the band assembly 2 comprises a front band 3, comprising two front band ends 3a, an upper band 4 comprising two upper band ends 4a and two side bands 5, each comprising a first side band end 5a and a second side band end 5b.

Each band 3, 4, 5 comprises an inner surface in contact with a portion of the user's head, and an outer surface. The terms "longitudinal" is used to describe the bands 3, 4, 5 according to their edges with a greater length, "transversal" is used to describe the bands according to their edges with a smaller width.

In use position, namely when the user wears the wearable device 1, the front band 3 has its inner surface that is placed against the forehead of the user. The upper band 4 has its inner surface that is placed against the top of the user's head and the side bands 5 have their inner surfaces that are respectively placed against the sides of the user's head. The second side bands ends 5b are placed against the occipital region of the user's head.

In the following paragraphs, only one side, including a side band 5, a half of the upper band 4 and a half of the front band 3, of the wearable device 1 is described considering that what is described for one side of the wearable device 1 is the same for the other side, except if it is specified that the wearable device 1 is described as a whole.

In this embodiment, the upper band end 4a is assembled to the first side band end 5a of the side band 5. The upper band 4 and the side band 5 are assembled near the parietal region of the user's head. When considering the whole wearable device 1, the structure 15 formed by the side bands 5 and the upper band 4 is continuous from one second side band end 5b to the other and generally presents a semicircular shape, or an oval shape. In one embodiment, the second side band ends 5b of each side band 5 are not in contact.

The width of the structure 15 gradually increases from the second side band ends 5a to the upper band 4. The great width of the upper end 4, compared to the side band 5 and the front band 3, maintain the wearable device 1 on the user's head.

The side band 5 is for example generally curve shaped to adapt to the side of the user's head.

The front band 3 may generally be circular such that it complies with the forehead shape of the user in use position.

The front band 3 is for example assembled to the side band 5 by the front band end 3a, approximatively at equal distance from the first and second side band ends 5a, 5b.

In an embodiment, the front band 3, the upper band 4 and the side bands 5 are made with a flexible thermoplastic material, such as polyurethane plastic or silicone rubber. The bands 3, 4, 5 are for example molded and form one single piece.

View from its side, the wearable device 1 is generally Y-shaped. More precisely, in a side view, the front band 3 forms a branch of the Y, the upper band 4 and at least the portion near the first side band end 5a of the side band 5 form the other branch of the Y, while at least the portion near the second side band end (5b) of the side band 5 forms the foot of the Y.

In this embodiment, the front band 3 may form an angle between 30° and 50° with the side band 5. Preferably, the front band 3 forms and angle of 40°.

To perform the control of the cerebral activity of the user during his sleep, the wearable device 1 may comprise embedded electronics 10. The embedded electronics 10 is for example disposed on or housed within the upper band 4.

The embedded electronics 10 may comprise at least a processor, a communication device and a battery. The communication device may comprise Bluetooth, WiFi or other wireless communication means for communication with one or more computing devices, such as mobile phone, laptop computer, desktop computer, tablet computer or a server.

The embedded electronics 10 may also include one or several ports 11 for connecting the wearable device 1 to a power source of charging. The port 11 may also be sued for interfacing the wearable device 1 with another computing device.

The embedded electronics 10 may be fixed on an electronic plate disposed within the upper band 4. To receive the embedded electronics 10, the upper band 4 may be thicker than the side bands 5 and front band 3. The side bands 5 and the front band 3 are quite thin such that the wearable device may still have a compact size.

To improve the comfort of the user, some pads 12 may be provided, at least on the inner surface of the upper band 4 which is in contact with the user's head.

The front band 3 may also comprise at least one sensor 7 such that when the wearable device 1 is in use position, the sensor 7 is in contact with the forehead of the user, as shown figure 2. The sensor may measure bioelectric physiological signals from the user.

At least one electrode 8 may also be provided on the side band 5. The electrode 8 may be supported by a support 9. In this embodiment, the support 9 is placed near the second side band end 5a. The support 9 may be empty such that the the adjustment element 13 is able to pass through the support 9.

The support 9 may be attached to the side band 5 for example by gluing it to the side band 5.

Alternatively, the support 9 may be removable such that the user can change the support 9 or replace it.

The electrode 8 is attached to the support 9 by any adapted fixing mean. For example, the electrode 8 comprises a plug 20 that slides into the support 9 to attach the electrode 8 to the band assembly 1. The plug is for example made with a conductive material such that the signals measured by the electrodes can be transmitted through the plug to the embedded electronics 10 by means of the physical components 17. The plug 20 is for example a metal plate. When the wearable device 1 is in use position, the electrode 8 is in contact with the rear part of the user's head, more precisely with the occipital portion of the head.

Alternatively, the electrode 8 may be removable from the support 9, as well as the sensor 7 can be removable from the front band 3.

In another embodiment, the electrode 8 and the support 9 form a whole, such that the body of the electrode 8 forms the support 9 and comprises the plug 20. In this embodiment, the electrode 8 can also be removable.

Each electrode 8 and/or sensor 7 may be electrically connected to the embedded electronics 10 disposed on or housed within the upper band 4. The embedded electronics 10 can be configured to receive signals from the electrode 8 and/or sensor 7 and provide an output to the processor. The embedded electronics 10 may be configured to perform at least some processing of the signals received from the electrode 8 and/or sensor 7. For example, the embedded electronics 10 may comprise a converter, and a processing system.

In one embodiment, the embedded electronics 10 is electrically connected to each electrode 8 and/or sensor 7 by one or more physical components 17, for example wires, extending between the embedded electronics 10 and each electrode 8 and/or sensor 7 inside the bands 3, 4, 5, such that the physical components 17 are invisible and inaccessible to the user.

In another embodiment, the physical components 17 electrically connecting the electrode 8 and/or sensor 7 to the embedded electronics 10 are embedded within the material forming the band assembly 2, i.e. in the bands 3, 4, 5.

The physical component 17 can be relied to the electrode 8 via the plug 20 made of conductive material.

The electrical connection of the electrode 8 and/or sensor 7 to the embedded electronics 10 inside the wearable device 1 provides a more compact design and protects the physical components 17 extending between the electrodes and the embedded electronics.

Alternatively, the bands 3, 4, 5 may be made of a conductive material such that no added physical components 17 are necessary to electrically connect the electrode 8 and/or the sensor 7 to the embedded electronics 10.

Otherwise, the wearable device 1 advantageously comprises an adjustment element 13 for adjusting the diameter of the head of the user. It particularly allows an adjustment of the wearable device 1 to the user's head and a good contact between the user's skin and the sensor 7 and the electrode 8.

The adjustment element 13 allows a modification of the dimensions of the band assembly 2 depending of the diameter of the head of the user to fit with said diameter.

In one embodiment, the adjustment element 13 may be made in a flexible and extendable material. For example the adjustment element 13 is an elastic ribbon made of elastomer or extendable cloth.

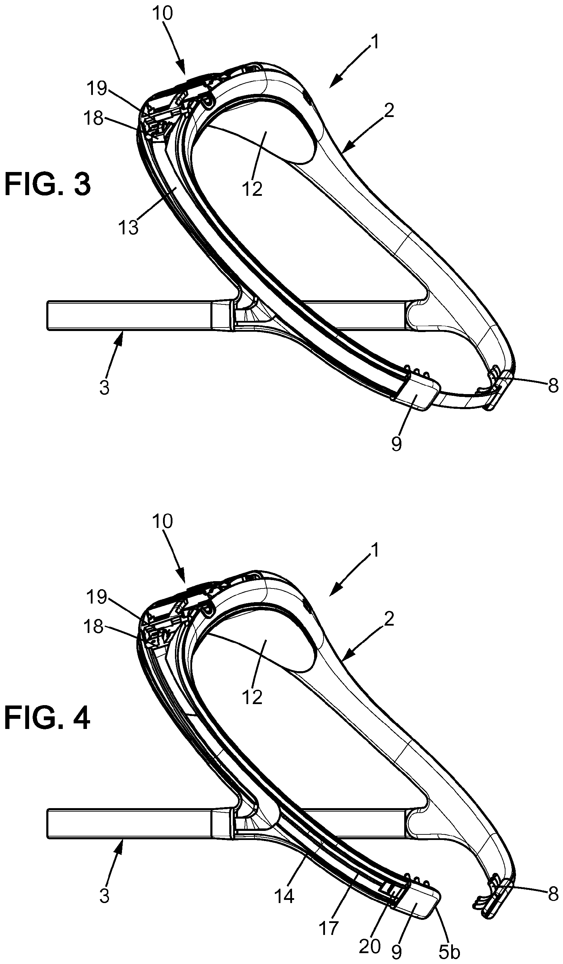

Referring to figure 3 and considering the wearable device 1 in its entirety, the adjustment element 13 comprises two ends 13a. Each end 13a is attached at the first side band end 5a of a side band 5.

For example, the adjustment element 13 can comprise a hole 18, at each of its ends 13a, in which a cleat 19, provided at the junction point of the upper band 4 and the side band 5, is inserted. The mechanical force exerted on the adjustment element 13, when worn by a user, may prevent the exit of the cleat 19 out of the hole 18.

The adjustment element 13 extends inside the side bands 5 from one of its end 13a to the other. To guide the adjustment element in the side bands 5, a channel 14 is provided in each of the side bands 5. The channel 14 may extend from the first end 5a to the second end 5b of each side band 5.

A portion of the adjustment element 13 is accessible between the two second side band ends 5a. The two side bands 5 can be moved away from each other since the second side band ends 5b are not in contact.

Hence, if the head of the user has a great diameter, the user can move apart the side bands 5 from each other to put the wearable device 1 on. The wearable device 1 adjusts to the head of the user by the mean of the adjustment element 13. The adjustment element 13 is able to extend along its length when the wearable device 1 is pulled on the user's head.

The adjustment element 13 may allow the electrode 8 to push against the head of the user when the wearable device 1 is in use position.

It has been found that the longer the adjustment element 13 is, the more the pressure is equally reported along the head of the user. Then, no matter what the size of the user's head is, the pressure ranges exerted on the electrodes 8 and on the user's head is sensibly the same and is equitably distributed on the circumference of the user's head. Due to the pressure exerted by the adjustment element 13, the wearable device 1 stays in place on the user's head during his sleep.

The upper, forehead and side bands 3, 4, 5 may be made with a flexible material such as plastic, rubber or other flexible materials, such that, when a force is applied to the band assembly 2, the side bands 5 may be allowed to twist, flex or deform. For example, the force can be applied by a user by moving apart the side bands 5 from each other to put the wearable device 1 on. The force may also be applied by the head of the user when the adjustment element 13 is under pressure due to the size of the head. This force may cause the fall of the wearable device 1 from the head of the user because of the mechanical force exerted by the adjustment element 13 on the top of the side bands 5. We may provide a film 16 on each side band 5 to reinforce the rigidity of the side bands 5. The film 16 may be made with a rigid material such as plastic or metal. The film 16 is coated longitudinally on an outer surface of the side bands 5.

The film 16 offsets the force exerted by the adjustment element 13 such that the wearable device 1 may not fall from the head of the user and still the side bands 5 can be moved apart from each other such that the wearable device 1 is able to adapt to the diameter of the user's head.

### References :

wearable device 1
band assembly 2
front band 3
front band end 3a
upper band 4
upper band end 4a
side band 5
first side band end 5a
second side band end 5b
sensor 7
electrode 8
support 9
embedded electronics 10
port 11
pad 12
adjustment element 13
end 13a
channel 14
structure 15
film 16
physical component 17
hole 18
cleat 19

## Claims

1. Wearable device (1) able to be worn on a user's head for controlling the cerebral activity of a user during sleep comprising a band assembly (2) comprising
- a front band (3), generally curve shaped, able to be placed against a forehead of the user, comprising two front band ends (3a);
- an upper band (4) generally curve shaped, able to be placed against a top head of the user, comprising two front band ends (4a);
- a first and a second side bands (5), each of said side bands (5) comprising
∘ a first side band end (5a) connected to an upper band end (4a) and a second side band end (5b) placed against the occipital region of the user's head,
∘ a channel (14) extending between the first and second side band ends (5a, 5b) able to guide an adjustment element (13), said adjustment element (13) comprising two ends (13a), each end (13a) being attached at the first side band end (5a) of a side band (5), the adjustment element (13) extending inside the side band (5) from said first side band end (5a) of said first side band (5) to said first side band end (5a) of said second side band (5), wherein a portion of the adjustment element (13) is accessible between the two second side band ends (5b), and
∘ at least one electrode (8) able to be in contact with the user's head,
each side band (5) being connected to a front band end (3a).

2. Wearable device (1) according to claim 1, wherein the side bands (5) and the upper band (4) are able to form a structure (15) having a semicircular shape able to encircle at least a portion of a circumference of the user's head.

3. Wearable device (1) according to any of claims 1 and 2, wherein the second side band ends (5b) of the first and second side bands (5) do not touch each other such that a portion of the adjustment element (13) is accessible between the second side band ends (5b) of said first and second side bands (5).

4. Wearable device (1) according to any of claims 1 to 3, wherein the front band (3) forms an angle between 30° and 50° with the side bands (5), preferably an angle of 40°.

5. Wearable device (1) according to any of claims 1 to 4, wherein the front band ends (3a) are disposed at equidistance from first and second side band ends (5a, 5b) of the side band (5) the front band ends (3a) are connected to.

6. Wearable device (1) according to any of claims 1 to 5, **characterized in that** the wearable device (1) has a sideway generally Y-shaped.

7. Wearable device (1) according to any of claims 1 to 6, wherein the adjustment element (13) comprises two ends (13a), each end (13a) being attached to a cleat (19) disposed at the first end (5a) of first and second side bands (5).

8. Wearable device (1) according to any of claims 1 to 7, wherein the adjustment element (13) is able to extend along its length when the wearable device (1) is pulled tight on the user's head.

9. Wearable device (1) according to any of claims 1 to 8, wherein the side bands (5) are made with a flexible material such that the side bands (5) are able to adapt to the shape of the user's head.

10. Wearable device (1) according to any of claims 1 to 9, wherein an outer surface of the side bands (5) is coated with a rigid film (16).

11. Wearable device (1) according to any of claims 1 to 10, wherein each electrode (8) is disposed on a support (9) placed at the second side band end (5b) of each side band (5) such that the electrode (8) is in contact with the rear part of the user's head when the wearable device (1) is worn.

12. Wearable device (1) according to any of claims 1 to 11, wherein the electrodes (8) are attached to the side bands (5) by a plug (20) made of conductive material.

13. Wearable device (1) according to any of claims 1 to 12, wherein the support (9) is comprised in the electrode (8).

14. Wearable device (1) according to any of claims 1 to 13, wherein the upper band (4) comprises embedded electronics (10).

15. Wearable device (1) according to any of claims 1 to 14, wherein the adjustment element (13) is an elastic ribbon.

## Patentansprüche

1. Tragbare Vorrichtung (1), die fähig ist, auf dem Kopf eines Benutzers getragen zu werden, um die Hirnaktivität eines Benutzers während des Schlafs zu steuern, die eine Gurtanordnung (2) aufweist, die aufweist:
- einen vorderen Gurt (3), der im Allgemeinen gekrümmt geformt ist, fähig ist, gegen die Stirn des Benutzers angelegt zu werden, der zwei vordere Gurtenden (3a) aufweist;
- einen oberen Gurt (4), der im Allgemeinen gekrümmt geformt ist, fähig ist, gegen den oberen Kopf des Benutzers angelegt zu werden, der zwei vordere Gurtenden (4a) aufweist,
- einen ersten und einen zweiten Seitengurt (5), wobei jeder der Seitengurte (5) aufweist:
∘ ein erstes Seitengurtende (5a), das mit einem oberen Gurtende (4a) verbunden ist, und ein zweites Seitengurtende (5b), das gegen die Hinterkopfregion des Kopfs des Benutzers angelegt ist,
∘ einen Kanal (14), der sich zwischen den ersten und zweiten Seitengurtenden (5a, 5b) erstreckt, der fähig ist, ein Einstellelement (13) zu führen, wobei das Einstellelement (13) zwei Enden (13a) aufweist, wobei jedes Ende (13a) an dem ersten Seitengurtende (5a) eines Seitengurts (5) befestigt ist, wobei sich das Einstellelement (13) innerhalb des Seitengurts (5) von dem ersten Seitengurtende (5a) des ersten Seitengurts (5) zu dem ersten Seitengurtende (5a) des zweiten Seitengurts (5) erstreckt, wobei ein Abschnitt des Einstellelements (13) zwischen den zwei Seitengurtenden (5b) zugänglich ist, und
∘ wenigstens eine Elektrode (8), die fähig ist, in Kontakt mit dem Kopf des Benutzers zu sein, wobei jeder Seitengurt (5) mit einem vorderen Gurtende (3a) verbunden ist.

2. Tragbare Vorrichtung (1) nach Anspruch 1, wobei die Seitengurte (5) und der obere Gurt (4) fähig sind, eine Struktur (15) mit einer halbkreisförmigen Form zu bilden, welche fähig ist, wenigstens einen Abschnitt eines Umfangs des Kopfs des Benutzers zu umgeben.

3. Tragbare Vorrichtung (1) nach einem der Ansprüche 1 und 2, wobei die zweiten Seitengurtenden (5b) der ersten und zweiten Seitengurte (5) einander nicht berühren, so dass ein Abschnitt des Einstellelements (13) zwischen den zweiten Seitengurtenden (5b) der der ersten und zweiten Seitengurte (5) zugänglich ist.

4. Tragbare Vorrichtung (1) nach einem der Ansprüche 1 bis 3, wobei der vordere Gurt (3) mit den Seitengurten (5) einen Winkel zwischen 30° und 50°, vorzugsweise einen Winkel von 40°, bildet.

5. Tragbare Vorrichtung (1) nach einem der Ansprüche 1 bis 4, wobei die vorderen Gurtenden (3a) in gleicher Entfernung zu ersten und zweiten Seitengurtenden (5a, 5b) des Seitengurts (5), mit denen die vorderen Gurtenden (3a) verbunden sind, angeordnet sind.

6. Tragbare Vorrichtung (1) nach einem der Ansprüche 1 bis 5, **dadurch gekennzeichnet, dass** die tragbare Vorrichtung (1) seitlich im Allgemeinen Y-förmig ist.

7. Tragbare Vorrichtung (1) nach einem der Ansprüche 1 bis 6, wobei das Einstellelement (13) zwei Enden (13a) aufweist, wobei jedes Ende (13a) an einer Klammer (19) befestigt ist, die an dem ersten Ende (5a) der ersten und zweiten Seitengurte (5) angeordnet ist.

8. Tragbare Vorrichtung (1) nach einem der Ansprüche 1 bis 7, wobei das Einstzellelement (13) fähig ist, sich entlang seiner Länge zu dehnen, wenn die tragbare Vorrichtung (1) auf dem Kopf des Benutzers festgezogen wird.

9. Tragbare Vorrichtung (1) nach einem der Ansprüche 1 bis 8, wobei die Seitengurte (5) mit einem flexiblen Material hergestellt sind, so dass die Seitengurte (5) fähig sind, sich an die Kopfform des Benutzers anzupassen.

10. Tragbare Vorrichtung (1) nach einem der Ansprüche 1 bis 9, wobei eine Außenoberfläche der Seitengurte (5) mit einer starren Dünnschicht (16) überzogen ist.

11. Tragbare Vorrichtung (1) nach einem der Ansprüche 1 bis 10, wobei jede Elektrode (8) auf einem Halter (9) angeordnet ist, der an dem zweiten Seitengurtende (5b) jedes Seitengurts (5) platziert ist, so dass die Elektrode (8) in Kontakt mit dem hinteren Teil des Kopfs des Benutzers ist, wenn die tragbare Vorrichtung (1) getragen wird.

12. Tragbare Vorrichtung (1) nach einem der Ansprüche 1 bis 11, wobei die Elektroden (8) durch einen aus einem leitfähigen Material hergestellten Stecker (20) an den Seitengurten (5) befestigt sind.

13. Tragbare Vorrichtung (1) nach einem der Ansprüche 1 bis 12, wobei der Halter (9) in der Elektrode (8) enthalten ist.

14. Tragbare Vorrichtung (1) nach einem der Ansprüche 1 bis 13, wobei der obere Gurt (4) eingebettete Elektronik (10) aufweist.

15. Tragbare Vorrichtung (1) nach einem der Ansprüche 1 bis 14, wobei das Einstellelement (13) ein elastisches Band ist.

## Revendications

1. Dispositif pouvant être porté (1) apte à être porté sur la tête d'un utilisateur pour contrôler l'activité cérébrale d'un utilisateur pendant son sommeil comprenant un ensemble bande (2) comprenant
- une bande avant (3), généralement de forme incurvée, apte à être placée contre le front de l'utilisateur, comprenant deux extrémités de bande avant (3a) ;
- une bande supérieure (4) généralement de forme incurvée, apte à être placée contre le sommet de la tête de l'utilisateur, comprenant deux extrémités de bande avant (4a),
- des première et deuxième bandes latérales (5), chacune desdites bandes latérales (5) comprenant
- une première extrémité de bande latérale (5a) connectée à une extrémité de bande supérieure (4a) et une deuxième extrémité de bande latérale (5b) placée contre la région occipitale de la tête de l'utilisateur,
- un canal (14) s'étendant entre les première et deuxième extrémités de bande latérale (5a, 5b) aptes à guider un élément de réglage (13), ledit élément de réglage (13) comprenant deux extrémités (13a), chaque extrémité (13a) étant fixée au niveau de la première extrémité de bande latérale (5a) d'une bande latérale (5), l'élément de réglage (13) s'étendant à l'intérieur de la bande latérale (5) de ladite première extrémité de bande latérale (5a) de ladite première bande latérale (5) à ladite première extrémité de bande latérale (5a) de ladite deuxième bande latérale (5), dans lequel une partie de l'élément de réglage (13) est accessible entre les deux deuxièmes extrémités de bande latérale (5b), et
- au moins une électrode (8) apte à être en contact avec la tête de l'utilisateur, chaque bande latérale (5) étant connectée à une extrémité de bande avant (3a).

2. Dispositif pouvant être porté (1) selon la revendication 1, dans lequel les bandes latérales (5) et la bande supérieure (4) sont aptes à former une structure (15) présentant une forme semi-circulaire apte à encercler au moins une partie d'une circonférence de la tête de l'utilisateur.

3. Dispositif pouvant être porté (1) selon l'une quelconque des revendications 1 et 2, dans lequel les deuxièmes extrémités de bande latérale (5b) des première et deuxième bandes latérales (5) ne se touchent pas de sorte qu'une partie de l'élément de réglage (13) est accessible entre les deuxièmes extrémités de bande latérale (5b) desdites première et deuxième bandes latérales (5).

4. Dispositif pouvant être porté (1) selon l'une quelconque des revendications 1 à 3, dans lequel la bande avant (3) forme un angle compris entre 30° et 50° avec les bandes latérales (5), de préférence un angle de 40°.

5. Dispositif pouvant être porté (1) selon l'une quelconque des revendications 1 à 4, dans lequel les extrémités de bande avant (3a) sont disposées à équidistance des première et deuxième extrémités de bande latérale (5a, 5b) de la bande latérale (5) à laquelle les extrémités de bande avant (3a) sont connectées.

6. Dispositif pouvant être porté (1) selon l'une quelconque des revendications 1 à 5, **caractérisé en ce que** le dispositif pouvant être porté (1) présente une forme de Y généralement de biais.

7. Dispositif pouvant être porté (1) selon l'une quelconque des revendications 1 à 6, dans lequel l'élément de réglage (13) comprend deux extrémités (13a), chaque extrémité (13a) étant fixée à une pointe (19) disposée au niveau de la première extrémité (5a) des première et deuxième bandes latérales (5).

8. Dispositif pouvant être porté (1) selon l'une quelconque des revendications 1 à 7, dans lequel l'élément de réglage (13) est apte à s'étendre le long de sa longueur lorsque le dispositif pouvant être porté (1) est tendu sur la tête de l'utilisateur.

9. Dispositif pouvant être porté (1) selon l'une quelconque des revendications 1 à 8, dans lequel les bandes latérales (5) sont composées d'un matériau flexible de sorte que les bandes latérales (5) sont aptes à s'adapter à la forme de la tête de l'utilisateur.

10. Dispositif pouvant être porté (1) selon l'une quelconque des revendications 1 à 9, dans lequel une surface externe des bandes latérales (5) est revêtue d'un film rigide (16).

11. Dispositif pouvant être porté (1) selon l'une quelconque des revendications 1 à 10, dans lequel chaque électrode (8) est disposée sur un support (9) placé au niveau de la deuxième extrémité de bande latérale (5b) de chaque bande latérale (5) de sorte que l'électrode (8) est en contact avec la partie arrière de la tête de l'utilisateur lorsque le dispositif pouvant être porté (1) est porté.

12. Dispositif pouvant être porté (1) selon l'une quelconque des revendications 1 à 11, dans lequel les électrodes (8) sont fixées aux bandes latérales (5) par une prise (20) composée d'un matériau conducteur.

13. Dispositif pouvant être porté (1) selon l'une quelconque des revendications 1 à 12, dans lequel le support (9) est compris dans l'électrode (8).

14. Dispositif pouvant être porté (1) selon l'une quelconque des revendications 1 à 13, dans lequel la bande supérieure (4) comprend de l'électronique intégrée (10).

15. Dispositif pouvant être porté (1) selon l'une quelconque des revendications 1 à 14, dans lequel l'élément de réglage (13) est un ruban élastique.
